# EUROPEAN PATENT SPECIFICATION

(11) **EP 1 404 386 B1**
(45) Date of publication and mention of the grant of the patent: **21.04.2010**
(21) Application number: 02735564.3
(22) Date of filing: 14.05.2002
(51) Int. Cl.: A61L 26/00

(54) **Method of modifying an eye covering in the form of a contact lens through EHD with active ingredients**
Methode der Modifizierung von Augenverbänden in Form von Kontaktlinsen durch EHD mit aktiven Inhaltsstoffen
Méthode de modification d'un couverture d'oeil dans la forme d'une lentille de contact par EHD avec des ingrédients actifs

(30) Priority: 14.05.2001 GB 0111721
(43) Date of publication of application: 07.04.2004
(73) Proprietor: BATTELLE MEMORIAL INSTITUTE, Columbus, OH 43201-2693 (US); Keates, Richard, Greenwich, CT 06836 (US)
(72) Inventor: KEATES, Richard, Greenwich, CT 06836 (US); COFFEE, Ronald, Alan, Haslemere, Surrey GU27 1HA (GB); ESSEX-LOPRESTI, Johnathan, Electrosols Limited, Oxford OX4 4GA (GB); DAVIES, David Neville, Electrosols Limited, Oxford OX4 4GA (GB); WAN, Margaret, Oxford OX5 2JD (GB); POLLARD, Marie, Bowmanville, Ontario (CA)
(74) Representative: Clark, Jane Anne
(86) International application number: PCT/GB2002/002240
(87) International publication number: WO 2002/092142

(56) References cited:
- WO-A-00/35524
- WO-A-00/67694
- DE-A- 19 947 711
- US-A- 4 452 925
- US-A- 4 952 212
- US-A- 4 978 713
- US-A- 4 981 841

## Description

This invention relates to methods of modifying eye coverings in the form of contact lenses especially, but not exclusively, for use during and after eye surgery.

In recent years, surgical techniques have been developed that enable the refractive errors in the eye to be corrected by using an excimer laser to ablate corneal tissue. One way of carrying out this procedure is known as photo refractive keratectomy (PRK). PRK treats refractive errors by removal of tissue from the surface of the cornea so as to enable the eye to focus light more directly on the retina. The PRK procedure begins with tropical delivery to the eye of anaesthetic followed by removal of a precise amount of corneal tissue by using an excimer laser to ablate or vaporise the part of the corneal tissue to be removed. Because this procedure involves removal of some of the corneal epithelial layer, patients can experience considerable pain until the epithelium heals and covers the treated area. Although eye drops containing pain killers and antibiotics are effective in reducing both operative pain and risk of infection, it is very difficult to control dosage and it is usual for over or under application to occur which may detrimentally affect the healing time.

In PRK the epithelial layer regenerates over the cornea after surgery within about three days and reasonable vision usually results within about seven days. In an attempt to reduce pain and healing time, other techniques have been developed in which, instead of removing the epithelial layer, the epithelial layer is treated with alcohol, peeled back as a hinged flap to expose the underside of the cornea for laser ablation and then laid back. This procedure is more comfortable than PRK but is cumbersome and has a slower visual recovery.

WO00/67694 describes quick dissolving tablets or pills formed of electrohydrodynamically produced fibres primarily for oral delivery but does suggest that they could be used for other purposes, for example to release a drug onto the eyeball surface after surgery. WO00/35524 describes in-situ delivery of EFET-produced droplets, short fibres or fibrils to the eye socket. US4952212 describes a method of administering a solution of an ophthalmically effective substance by supplying electrically charged droplets to the eye using electrostatic spraying apparatus. US4452925 describes hydrogels useful for contact lens manufacture where the contact lenses are formed using a moulding process. US4978713 describes the manufacture of a contact lens of a cross-linked organic aprotic solvent-insoluble polymer which is a derivative of polyvinyl alcohol where the contact lens is formed using a conventional spin-casting mould. US4981841 describes improvements in wound healing particularly after surgery such as a keratorefractive procedure, which involve placing a corneal mortar composition into the wound using, for example, a large bore needle or suitable trowel like tool. DE19947711 describes an implant for insertion in an opening in the eye in glaucoma therapy.

The present invention provides a method of modifying an eye covering in the form a contact lens, as set out in claims 1.

Usually, droplets, fibre and fibrils will be generally circular in cross-section but, if not, the diameter range should be taken as the equivalent circle diameter, that is the diameter of a circle having the same area as the fibre cross-section. Electrohydrodynamic processing (EHD) is described in, for example, GB-A-1569707, WO98/03267 and WO00/67694. The electrically charged nature of the resultant fibre, fibre fragments and/or droplets, (hereinafter referred to individually or collectively as "comminuted matter") results in a very even distribution of comminuted matter over a deposition surface.

An eye covering embodying the invention may be used as an eye bandage in contact with an exposed surface portion of an eye, for example after trauma or before, during or after surgery, for example, laser eye surgery such as PRK. Such an eye bandage may be used to reduce discomfort and pain caused by exposing cut nerve endings during the surgery and also to prevent further damage to the epithelial layer after the laser surgery. An eye covering embodying the invention carries an active ingredient for delivery to the exposed surface portion of the eye.

In an embodiment of the present invention the portion of the body comprising biologically compatible polymer fibre may carry the active ingredient to be delivered to the eye.

The active ingredient may be in the form of drugs, medicaments, biological molecules and the like. The active ingredient may be, for example, an anaesthetic, an antibiotic to reduce the risk of infection, a pain killer or analgesic (such as aspirin, ibuprofen, paracetomol) to reduce pain such as post-operative pain, an anti-bacterial, an anti-inflammatory, an anti-viral, an anti-fungal antibiotic (such as chloramphenicol, gentamicin and ciprofloxacin), a growth factor such as hepatocyte or epidermal growth factor to promote epithelial cell growth during the healing process and/or a phospholipid surfactant such as a surfactant protein or lecithin which may also enhance the healing of the epithelial layer. Other factors may be delivered to reduce inflammation. The use of EHD processing to produce the biologically compatible polymer fibre enables precise control and distribution of the active ingredients so enabling precise control of the dosage delivered to the eye. Moreover, because the eye covering is pre-formed it is not necessary for a surgeon to spray electrohydrodynamically processed comminuted matter directly into the patient's eye.

The biologically compatible polymer fibre may be sprayed onto the contact lens after manufacture and immediately before packaging of the contact lens in a hematically sealed package.

The polymer may be selected from, for example, collagen, polylactide, 2-hydroxyethylmethacrylate and polyethylene oxide.

An eye covering is described which comprise or include fibres of a non-biodegradable non-aqueous based polymers, for example, polyvinyl acetate, nitrocellulose, polyvinylchloride. In this case, the eye covering will need to be removed in due course.

As another possibility, an eye covering for contacting an exposed surface portion of an eye may be formed from polymer fibres consisting of one or more types of polymer, natural or synthetic, for example any one or more of the polymers mentioned above.

The polymer fibre layers such as HEMA and PEO fibre layers may provide adhesive layers that facilitate adhesion to the eye while the non water-soluble polymer fibres mentioned above should provide increased strength during use.

The polymer fibre may carry the active ingredient (within the polymer fibre or sprayed onto the polymer fibre) to be delivered to the eye enabling, as described above, precise targeted delivery of a medicament or other material to the eye.

The active ingredient may be any of the ingredients mentioned above such as a pain killer or analgesic, an antibiotic, an anti-inflammatory, another medicament, a biological molecule such as a growth hormone and so on. Where the active ingredient is incorporated into the fibre, then the liquid formation from which the fibre is produced may comprise a fully dissolved solution of the active ingredient, a suspension or a microemulsion, for example.

The use of polymer fibre to form the eye covering has further advantages in that the strength-to-weight ratio of the eye covering should be greater than that of a conventional eye bandage or contact lens. Thus, the polymer fibre eye covering may be made very thin for example less than 200 micrometers thick, typically 15 to 70 micrometers thick, and this, combined with the relative porous nature of the fibre network, means that the eye covering is very light in comparison to a conventional contact lens and should therefore stay in position on the exposed surface portion of the eye better than would a conventional contact lens. Indeed, such an eye covering may be sufficiently light in weight that pre-shaping to conform to the exposed surface portion may not be necessary in order for the eye covering to stay in place.

Typically, the eye covering will be circular having a diameter sufficient just to cover the injured epithelial layer and cornea for example 9 to 15 mm (just enough to cover the epithelial layer which is about 8mm in diameter). Larger or smaller eye coverings may, however, be provided, where appropriate.

An eye covering as set out above is described which is hermetically sealed within a blister pack or capsule.

In an embodiment, the present invention provides a conventional contact lens such as a hydrogel contact lens, wherein at least a proportion of the contact lens for example the periphery, is structurally reinforced by a thin coating of polymer fibre deposited onto the contact lens using EHD processing. As used herein the term "hydrogel" refers to a water soluble (hydrophilic) polymer that maintains some sort of solid structure when exposed to water. Such reinforcement of the contact lens should reduce the possibility of damage, in particular should increase the resistance of the contact lens to shear tearing, and so should facilitate handling during use. The thin coating may be of a water-soluble polymer fibre that dissolves in the eye so that, once the contact lens has been correctly positioned in the eye, the reinforcement disintegrates or dissolves, enabling the contact lens proper to be thinner than is usually possible which should provide greater comfort in use. As another possibility the reinforcing polymer fibre may be biodegradable or non-biodegradable.

As used herein the term "biologically compatible polymer" means any polymer, synthetic or natural, that can be applied to or deposited onto an exposed surface portion of an eye without any unintended adverse or any unintended significant adverse effect.

As used herein, the term "active ingredient" includes any material that has an effect, generally not an adverse effect, on the eye or its environment, for example, drugs or medicaments such as analgesics, pain killers, biological molecules and so on.

As used herein the term "biologically degradable polymer" means that the polymer degrades, disintegrates or dissolves when used for its intended purpose, that is when placed on an exposed surface portion of the eye, within a relatively short period of time, for example, a matter of a few days, commensurate with the length of time normally required for healing subsequent to surgery on or trauma to the eye.

Embodiments of the present invention will now be describe, by way of example, with reference to the accompanying drawings, in which:
Figure 1 shows a very simplified diagrammatic representation of electrohydrodynamic processing apparatus for producing eye coverings embodying the present invention;
Figure 2 shows a very diagrammatic representation of a former suitable for use in the apparatus shown in Figure 1 ;
Figures 3 and4 show, very diagrammatically, a front view and a cross sectional view through an eye covering embodying the present invention; and
Figure 5 shows a cut-away diagrammatic representation of a blister pack incorporating an eye covering embodying the present invention.

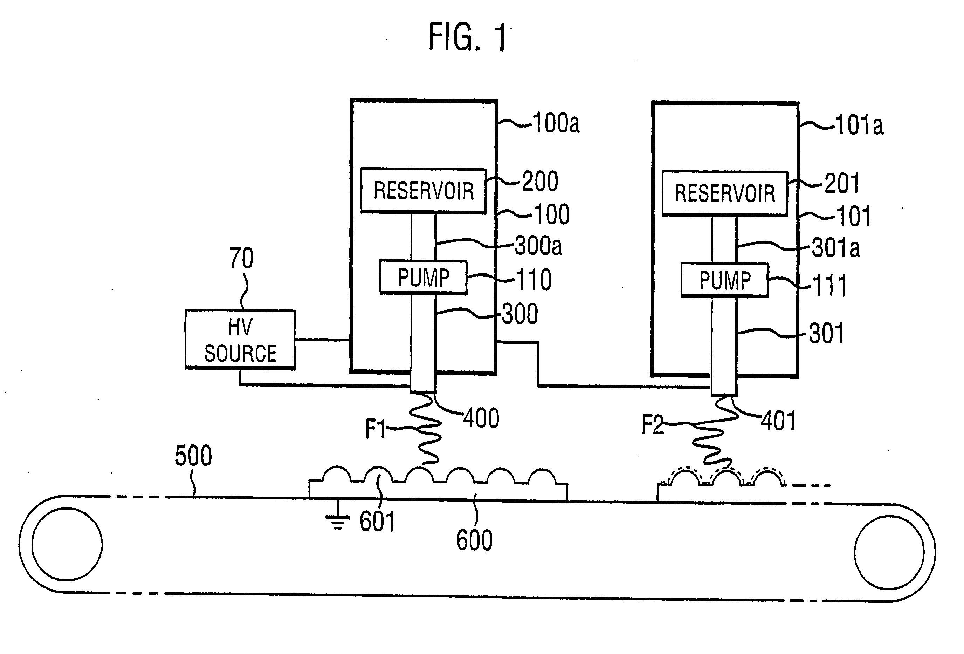

Referring now to the drawings, Figure 1 shows very diagrammatically one example of an electrohydrodynamic processing (EHD) device 1 with a housing 1a of the device 1 cutaway to show functional components of the device.

The housing 1a contains a reservoir 2 of liquid formulation to be subject to EHD processing. The reservoir 2 is coupled via a supply pipe 3a to a pump chamber 10 which is itself coupled to pump liquid into a supply tube 3 having an outlet 4.

In this example, the supply tube 3 is formed of an electrically insulative material. A first electrode 5a is mounted within the electrically insulative tube 3 and a second electrode 5b is mounted to the exterior of the electrically insulative tube 3.

The outlet 4 is positioned adjacent a housing outlet 1b to enable comminuted matter to be dispensed from the EHD device 1 as will be described below.

The housing also contains a voltage source (6 such as a battery), which is coupled via a switch SW1, generally a push button switch mounted to the housing 1a, to a high voltage generator 7 and to the pump 10.

The high voltage generator 7 may be, for example, an electromagnetic high voltage multiplier of the type supplied by Brandenburg, Astec Europe of High Street, Wollaston, Stourbridge, West Midlands DE8 4PG, UK, or Start Spellman of Unit 1, Broomers Park, Broomers Hill Lane, Pullborough, West Sussex, RH20 2RY, UK. As an alternative, a piezoelectric high voltage source which has a low capacitance may be used. Typically, the high voltage generator generates a voltage of several kilovolts for example a voltage in the range of 10 to 20 kilovolts.

In this example, the EHD device 1 is designed so as to be used by an ophthalmic surgeon before, during or after laser eye surgery such as PRK type laser eye surgery and the housing 1 is sized and shaped so as to be grasped easily in the hand.

The reservoir 2 contains a liquid formulation comprising a biologically compatible natural or synthetic polymer carrying an active ingredient.

In this example, the EHD device 1 is intended to be used by the surgeon after PRK or similar laser eye surgery and accordingly the active ingredient comprises at least one of a pain killer, an antibiotic and a growth factor such as hepatocyte growth factor for prompting epithelial cell growth during healing. Other active ingredients that may be incorporated include anti-inflammatories. The manner in which the active ingredient is carried by the liquid formulation will depend upon the particular formulation and active ingredient. For example, the active ingredient may be fully dissolved in the liquid formulation, may be provided in suspension in a liquid formulation or in microsuspension or as the microemulsion within the liquid formulation.

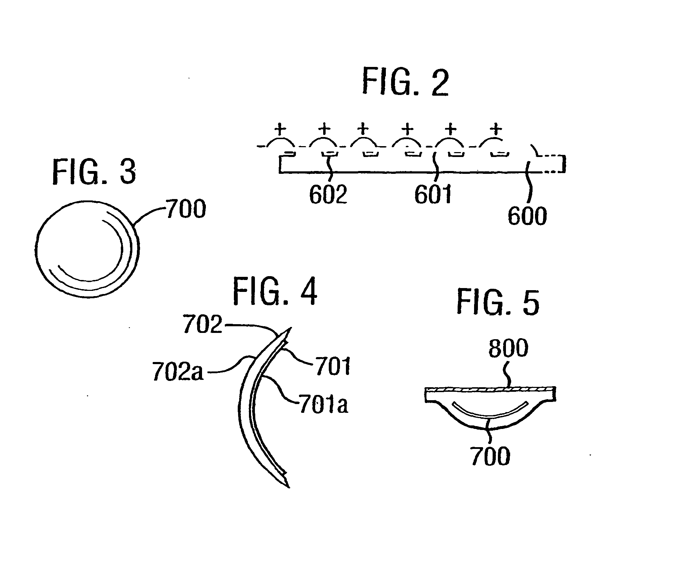

In this example, once the surgeon has carried out the PRK or similar laser treatment, that is the desired amount of corneal tissue has been ablated using an excimer laser, then the surgeon grasps the EHD device 1 in his hand 8 so that, as shown in Figure 2, the outlet 1b of the housing 1a is positioned over the portion 20a of the eye surface 20 exposed during the laser eye surgery and then activates the switch SW1 using, for example, a finger. Activation of the switch SW1 couples the voltage source 6 to the high voltage generator 7 and thus pump 10 so that liquid is pumped to the outlet 4 and liquid issuing from the outlet is subjected to the high electric field generated by the high voltage generator 7 causing the liquid to form a jet. As described with reference to Figures 2a to 2c of WO 98/03267, dependent upon the liquid formulation and the flow rate to the outlet 4, the liquid jet will form electrically charged comminuted matter which will comprise at least one of electrically charged fibre, fibre fragments ("fibrils") and droplets. As shown in Figure 2, the EHD processing results in formation of a fibre F.

Because the comminuted matter is electrically charged, it deposits uniformly and evenly over the exposed surface portion 20a of the eye so enabling uniform delivery of the active ingredient over the exposed surface portion 20a of the eye. In addition, because generation of the comminuted matter can be controlled easily by activating the switch SW1 the surgeon can control accurately the length of time for which comminuted matter is delivered to the exposed surface portion 20a of the eye and can thus control the dose of active ingredient received by the exposed surface portion 20a.

The EHD device 1 thus enables accurate and controlled delivery of an active ingredient to the exposed surface portion 20a to enable reduction of discomfort and pain caused by exposure of cut nerve endings during, for example, PRK laser eye surgery. Where the liquid formulation results in fibre formation as shown in Figure 2, then the fibres will deposit upon to the exposed surface portion 20a of the eye to build up a thin translucent layer or eye bandage in contact with the exposed surface portion. In this case, in addition to delivering an active ingredient to reduce discomfort and pain, the fibre bandage protects the exposed surface portion 20a against further damage to the epithelial layer and should also provide a physical barrier to reduce the possibility of post-operative infection. While, because of the porous nature of the fibre layer, still allowing air, eye drops and drugs to pass through so that good epithelium health can be maintained. Typically the liquid formulation and flow rate will be selected such that the fibres have a diameter up to several hundred micrometers, preferably in the range 1 to 10 micrometers.

In this example, the liquid formulation comprises a solution of polylactide in a suitable solvent such as acetone or ethyl acetate. The use of polylactide, has the advantage that the resultant polymer fibres are biodegradable with a half life comparable to the time of normal healing after PRK laser eye surgery, for example, within two to three days, so that over that time period the fibres gradually disintegrate or dissolves away. This has the advantage that removal of the fibre eye bandage or dressing is not necessary. Other biologically compatible biodegradable polymers may also be used to produce such an *in situ* polymer fibre eye bandage or dressing such as polyglycolide, polylactide-co-glycolide, polycaprolactone, polylactide-co-caprolactone, Eudragit^{™}(a co-polymer of acrylic and methyacrylic acid esters), Biopol^{™} (a co-polymer of hydroxybutyrate and hydroxyvalerate).

In the above described example, the comminuted matter produced by the EHD device 1 is deposited directly onto the exposed surface portion 20a of the eye 20.

Figure 3 shows a diagram for explaining eye covering or bandage embodying the present invention. In this case, a conventional contact lens manufactured by a conventional contact lens manufacturing process such as described in, for example, Reports of Patent Design and Trade Mark Cases (RPC) 1997, No. 9 at pages 305, 306 and 350 to 361 is provided.

A method of manufacturing or preforming an eye covering for delivering active ingredient to an exposed surface portion of an eye will now be described with the help of Figures 1 to 3.

Figure 1 shows a much simplified very schematic diagram of apparatus that may be used in the manufacture of the eye covering. In this case, two EHD devices 100 and 101 are provided. These devices are intended to be mounted to a gantry or other support (not shown in Figure 1). Each EHD device 100, 101 comprises a housing 100a, 101a containing a reservoir 200, 201 that supplies liquid formulation via a liquid supply pipe 300a, 301a to a pump 110, 111 coupled to, in this case, an electrically conductive supply tube 300, 301, which projects from an outlet of the housing 100a, 101a of the device. In this case, an external high voltage source 70 is provided that is coupled to each of the electrically conductive tubes 300, 301.

The EHD devices 100, 101 are mounted above an electrically conductive support surface 500 which, in this example, is coupled to earth (ground). As shown, the suppose surface 500 is in the form of a movable electrically conductive conveyer belt. The surface 500 supports a number of preferably electrically conductive formers 600 each of which defines an array of protrusions 601 each of which is shaped to mimic the surface portion of an eye to which the eye covering is to be applied.

In use, the EHD devices 100 and 101a are activated to produce electrically charged fibre F1, F2 and the movable conveyer belt 500 is moved so as to pass the former 600 beneath first one and then the other of the EHD devices 100 and 101 so that successive layers of polymer fibre F1 and F2 are built up upon the former 600 to form the eye coverings. The high voltage generator may be arranged to cause the fibres F1 and F2 to be oppositely charged so as to facilitate deposition of one upon the other.

The layers of fibre may build up over the entire surfaces of the formers 600, in which case a cutting device such as a knife or laser may be used to separate the individual eye coverings formed on the respective protrusions 601. As another possibility, as shown diagrammatically in Figure 2 and as described in WO 00/67694 with respect to Figure 5 of that document, the formers 600 may be arranged so that the protrusions 601 carry a charge opposite to that of the fibres F1 and F2 while the islands 602 between the protrusions 601 may carry the same charge as the fibres F1 and F2 so that the electrically charged fibres are repelled from the islands 602 and attracted to the protrusion 601. In this case, little or no subsequent separation of the eye coverings should be necessary.

Figures 3 and 4 show a front plan view and a cross-sectional view of a resulting eye covering. As can be seen from Figure 3, the eye covering 700 is generally circular disc-like in shape and, as can be seen from Figure 4, comprises first and second polymer fibre layers 701 and 702. By virtue of the deposition on the former 601, a rear surface 701a of the eye covering formed by the polymer layer 701 is concave and has a shape that is designed to conform to the exposed surface portion 20a of the eye while the front surface 702a formed the by the polymer fibre layer 702 is outwardly convex.

Generally, the disc-like eye covering will have a diameter comparable to that of a conventional contact lens, for example, 9 to 15 millimetre and may be designed just to cover the injured epithelial layer and cornea.

The fibre layers 701 and 702 may be deposited for a time period such that the overall thickness of the resulting eye covering is less than 200 micrometers, typically 50 to 70 micrometers.

After manufacture, the eye coverings 700 may be individually hematically sealed in blister packets 800 as shown in Figure 5 in a manner similar to conventional disposable contract lenses, preserved in an appropriate preserving sterile environment.

Examples of particular liquid formulations for producing particular polymer fibre layers will now be described.

In one example, one of the two polymer fibre layers 701 and 702 of the eye covering 700 is formed of hydrophilic polymer fibre and the other of the two polymer fibre layers is formed of water-based polymer fibres as collagen, chondroitin sulfate, gelatin, gum arabic, hydroxypropylcellulose, hydroethylcellulose, hydrpxypropy-1 methyl cellulose, carboxymethyl cellulose, Eudragit^{™} S100 (a co-polymer of methyacrylic acid and methylmethacrylate), polyvinyl alcohol (PVA), polyvinyl pyrrolidone (PVP), 2-hydroxyethylmethacrylate , polyethylene oxide. The liquid formulations used to produce these polymer fibres may consist of the polymer dissolved in a suitable solvent which will depend upon the polymer but may be an aqueous solvent, ethanol or an ethanol water mixture or a melt. Either or both of the liquid formulations may contain active ingredient. The active ingredient may be any or more of drugs, medicaments, biological molecules and the like. The active ingredient may be, for example, an anaesthetic, an antibiotic to reduce the risk of infection, a pain killer or analgesic (such as aspirin, ibuprofen, paracetomol) to reduce pain such as post-operative pain, an anti-bacterial, an anti-inflammatory, an anti-viral, an anti-fungal antibiotic (such as chloramphenicol, gentamicin and ciprofloxacin), a growth factor such as hepatocyte or epidermal growth factor to promote epithelial cell growth during the healing process, and/or a phospholipid surfactant such as a surfactant protein or lecithin which may also enhance the healing of the epithelial layer. Other factors may be delivered to reduce inflammation. The active ingredient may be in solution, in suspension, in microsuspension, in emulsion or in microemulsion.

Where, in this example, the second or outer polymer fibre layer 702 is formed of a water-soluble polymer such as PVP or PVA, the purpose of this layer is primarily to strengthen the eye covering to reduce the possibility of damage during the process from removal from the blister pack 800 to placement on the exposed eye portion. In this case, when the eye covering is placed on the exposed eye portion 20a, the presence of tears will cause the water-soluble polymer fibre layer to dissolve virtually instantly leaving the hydrophilic polymer fibre layer in place on the eye. The water-soluble polymer may, upon dissolving, disperse active ingredient into the eye.

The HEMA polymer fibre is used, it may already be hydrated, that is it forms a hydrogel. As another example, the hydrophilic polymer fibre layer may be deposited onto the former 600 in a non-hydrated form. In this case, when the eye covering is placed on the exposed eye portion, the water-soluble polymer fibre layer will again dissolve in the presence of tears and the hydrophilic polymer layer will swell to become a hydrogel that covers the epithelial surface.

As further possibilities, one or other of the fibre layers may be replaced by a polymer fibre layer comprising a biodegradable non-aqueous based polymer, for example polylactide, polyglycolide, polylaotide-co-glycolide, polycaprolactone, polylactide-co-caprolactone, Eudragit^{™} (a co-copolymer of acrylic and methacrylic acid esters), Biopol^{™} (a co-polymer of hydroxybutyrate and hydroxyvalerate) and/or a non-biodegradable non-aqueous based polymer, for example polyvinyl acetate, nitrocellulose, polyvinyl chloride. In each case, the liquid formulation will use a suitable solvent or may comprise a melt if a suitable solvent is not available.

In the above described examples, where a water-soluble polymer fibre layer is provided it forms the outermost layer of the eye covering. However, because the fibre layers are very thin, both the first deposited and the second deposited fibre layer will closely follow the shape of the former 601. Accordingly, the water-soluble polymer fibre layer may provide the rear surface 701a of the eye covering that contacts the exposed surface portion 20a in use, facilitating adhesion of the eye covering to the exposed surface portion.

As a further possibility, such an eye covering may comprise three of more polymer fibre layers, one of which may be a hydrophilic polymer layer and one of which may be a water-soluble polymer layer with the latter acting primarily to strengthen the eye covering before use and during application and possibly also providing a source of active ingredient to be dispersed onto the eye. One or more further ones of the polymer fibre layers may be biodegradable so that the fibres degrade or dissolve gradually with time slowly releasing the same or a different active ingredient to enable controlled or delayed delivery of the same or different active ingredient. As another possibility, the entirety of the eye covering may be formed of biodegradable polymer fibre that has a half life comparable with the healing process so that the eye covering disintegrates or dissolves with time and it is not necessary to remove the eye covering at the end of the healing period.

As another possibility, the eye covering 700 may be formed of fibres of a single type of biodegradable non-aqueous based polymer, for example polylactide, polyglycolide, polylactide-co-glycolide, polycaprolactone, polylactide-co-caprolactone, Budragit ^{™} (a co-copolymer of acrylic and methyacrylic acid esters), Biopol^{™} (a co-polymer of hydroxybutyrate and hydroxyvalerate) and/or a non-biodegradable non-aqueous based polymer, for example polyvinyl acetate, nitrocellulose, polyvinyl chloride.

In the above described examples, where water-soluble polymer is used the other polymer fibres or materials forming the eye covering should be sufficiently dry to avoid causing the water-soluble polymer fibres to dissolve or disintegrate prior to placement in the eye.

In the examples described above with reference to Figures 4 to 8, the entirety of the eye covering or bandage is formed of polymer fibres and active ingredient such as those described above may be incorporated into any one or more of the polymer fibre layers. As another possibility, droplets carrying active ingredient may be sprayed onto the polymer fibres during or after the deposition as described in, for example, WO 98/03267 or WO 00/67694.

In the above described examples, the polymer fibres are produced by EHD processing and deposited onto a surface, of an existing contact lens. The active ingredient may be sprayed onto the fibre as it is formed or deposited. In addition, the fibre and/or the polymer may be modified during flight or after deposition. For example, the polymers may be a side-chain modified polymer which is cross-linkable by ultra violet (UV) light, in which case the fibre may be exposed to UV light during flight and/or after deposition. Other forms of such processing may be used. For example, the polymer fibre may be subjected to a chemical environment, for example a gaseous environment, that causes a change, such as cross-linking, in the polymer fibre during the flight or after deposition. Where the polymer fibre is subject to modification after the deposition, then part of the deposited fibre may be masked. This would enable, for example, the shape of the eye covering to be defined by masking the deposited fibre and exposing only an unmasked area to a cross-linking or other modifying environment.

The unmodified polymer could then be selectively removed, for example washed away, leaving an eye covering of the desired shape.

As another possibility, droplets may be sprayed onto a deposited fibre layer using, for example, EHD spraying that reacts with the deposited fibres to cause hardening or cross-linking of the fibres only in a circular pattern area onto which the droplets are deposited. Again, the areas of the fibre mat onto which the droplets have not been deposited could be electively removed to leave the desired eye covering shape.

Of course, the above described masking procedures could be reversed, that in the undesired portion of the fibre layer could be modified by reaction with UV light, vapour, gas or droplets of another material to form a reaction product that is easily electively removed.

In the above described examples, one or more layers of a single type of polymer fibre are used to form the eye bandage or covering. As another possibility, one or more layers may be formed by simultaneous deposition of EHD produced polymer fibres formed of different polymers so that the or each layer of the eye covering or bandage consists of more than one polymer. For example, a layer may include different polymers selected for different characteristics such as strength, water-solubility and hydrophilic nature etc. Relative motion may be effected between the EHD processing devices and the target surface to enhance intermingling of the different fibres.

An eye covering embodying the invention may be applied to the eye prior to surgery to prepare the patient for surgery, for example such an eye covering may contain or carry an anaesthetic or numbing agent. This would have an advantage over prior conventional eye bandages in that a precise controlled delivery of the anaesthetic over the entire surface of the eye would be possible.

As mentioned above, non biodegradable hydrophobic polymer fibres such as PVC fibres may be used as the eye covering and then removed after a period by the surgeon.

In the above described examples, when the eye covering 700 is encapsulated in, for example, a blister pack or similar capsule, it may simply be hermetically sealed in sterile air. However, it will generally be desirable to incorporate some form of sterile retaining medium within the blister pack to prevent the eye covering 700 drying out, as is done for conventional disposable contact lenses. Where the polymer fibre eye covering includes a water-soluble polymer fibre layer then the conventional saline solution may be replaced by an appropriate liquid or other sterile environment such as a gaseous environment within which the water-soluble polymer fibre layer will not dissolve. Where is it not required that the water-soluble polymer dissolves or disintegrates in tears, ten the polymer may be cross-linked, for example by exposure to ultra-violet or a chemical that induces cross-linking during fibre formation or after deposition. In this case saline solution may be used.

In the above examples, described with reference to Figures 4 to 8, the entirety of the eye covering 700 is formed from polymer fibre layers and any active ingredient is incorporated into the polymer fibre. As another possibility as described in WO 00/67694, for example, the apparatus shown in Figure 4 may comprise one or more further EHD devices designed to generate comminuted matter in the form of droplets or fibrils carrying active ingredient which is deposited onto the fibre during or after deposition so that the droplets or fibrils stick to the fibre.

As a further possibility embodying the invention, a main body of the eye covering 700 is a conventional contact lens onto which one or more polymer fibre layers as described above are deposited. In this case, the polymer fibre layer may be deposited during manufacture after formation of the contact lens or, as another possibility, the polymer fibre layer or layers may be deposited onto a surface of the mould within which the contact lenses are to be formed.

In the above described examples, the eye covering is intended to protect the eye and/or to supply an active ingredient to the eye after laser eye surgery. The eye coverings described above may be used to protect the eye and/or deliver active ingredient after other forms of surgical procedure and in other cases were protection of the eye is required, for example, during healing of accidental eye trauma. In addition to delivering an active ingredient to reduce the discomfort and pain, an eye covering embodying the invention may protect an exposed surface portion of an eye against further damage to the epithelial layer and should also provide a physical barrier to reduce the possibility of post-operative infection, while, because of the porous nature of the fibre layer, still allowing air, eye drops and drugs to pass through so that good epithelium health can be maintained.

The pre-formed or pre-manufactured eye coverings described above are intended primarily for medical use by surgeons or like skilled personnel during treatment of an eye. This being the case, it is not generally necessary for the eye covering to have optical properties that correct the wearer's vision. It may, however, be possible to control deposition of the fibre layers and/or to shape the deposited fibre layers using a conventional lathing technique as described in the discussion in Reports of Patent Design and Trade Mark Cases (RPC) 1997, No. 9 at pages 305, 306 and 350 to 361 of a correctional contact lens manufacturing process to provide the eye covering with at least some rudimentary or crude optical characteristics to assist the wearer's vision during the treatment.

In the examples described above, the eye covering is intended to be used during medical treatment, for example after surgery or trauma to the eye. An EHD device as described above may, however, also be used to deposit polymer fibres onto a conventional contact lens, especially a disposable contact lens, to strengthen the contact lens and make it more resistant to shear tearing during handling by the wearer. For example, a water-soluble polymer material may be deposited around the periphery of the contact lens so that the periphery is strengthened during handling but the water-soluble polymer instantly or rapidly dissolves or disintegrates when in the presence of tears, that is when placed on the eye in normal manner. The water-soluble polymer maybe, any of those discussed above. Such conventional contact lenses may also be strengthened by applying a thin coat of a biodegradable polymer fibre such as polylactide, HEMA and PEO polymer fibres may also act as a gentle adhesive to stop the eye covering moving around.

In the above described examples, the EHD devices have a single outlet nozzle. The EHD device may, however, have a number of outlets or nozzles as described in WO 98/03267 enabling, for example, production of more than one fibre at a time. Also, in the above described examples, the polymer fibres are fibres of a single polymer. This need not necessarily be the case and for example, composite polymer fibres may be produced as described in WO 00/67694 with a core of the polymer fibre having different properties from its coating and possibly also carrying different active ingredients. As an example, an outer coating of the polymer fibre may comprise a water-soluble polymers fibre which disperses rapidly when the eye covering is placed in the eye leaving the polymer fibre core intact.

The eye coverings described above enable electrohydrodynamic processing to be used to deliver healing agents and analgesics to an exposed portion of an eye in controlled manner. As used herein, the terms "exposed portion" and "exposed surface portion" should be taken to include both the portion of the eye surface that is normally exposed, that is the conjunctiva, and also a surface exposed by trauma or during surgery, for example a corneal epithelial layer exposed during laser ablation of corneal tissue.

Where an EHD produced eye covering is to be used after surgery or trauma, then it may include at least one of droplets, fibrils and fibres of a biologically compatible polymer which degrade in the environment of the eye (for example', under the action of an enzyme in tears) over a period comparable to the normal healing period after such surgery or trauma.

In an embodiment described above, electrohydrodynamic processing is used to modify an existing conventional contact lens, for example by coating it with fibres, fibrils and/or droplets of an active ingredient to be supplied to the eye to enable good dosage control or for example to increase the strength, especially the resistance to shear tearing, of the contact lens.

In an embodiment described above, a composite eye covering comprising a contact lens is produced one face of which is water-soluble and dissolves in the environment of the eye and the other surface of which is water absorbant and, in the presence of tears or suitable eye drops, dissolves to form a hydrogel lens.

As discussed above, the eye covering carries one or more active ingredients.

Droplets of PVA or PVP may be sprayed by EHD processing directly onto an eye covering embodying the invention prior to, during or after application to the eye, for example for lubrication purposes.

The techniques disclosed in WO 00/67694 that enable slow or controlled release of drugs or medicaments may also be used.

Other forms of EHD devices than those described above may be used as described in, for example, GB-A-1569707, WO 98/03267 and WO 00/67694 for example at least in some circumstances the pump may be omitted and a gravity feed used.

Other biologically compatible polymers and polymer formulations than those described above may be used.

## Claims

1. A method of modifying an eye covering in the form of a contact lens, which method comprises positioning the eye covering adjacent an electrohydrodynamic comminution device comprising a liquid supply with an outlet and an electric field generator and activating the device so that liquid issuing from the outlet is subjected to an electric field causing the liquid to form at least one electrically charged jet which then forms at least one of electrically charged fibre, fibre fragments and droplets which deposit onto the eye covering,
wherein the liquid carries an active ingredient.

2. A method according to claim 1, wherein the active ingredient is selected from the group consisting of an antibiotic, a painkiller, an anti-inflammatory, a growth factor such as hepatocyte growth factor, an epidermal growth factor to promote epithelial cell growth during the healing process, and/or a phospholipid surfactant such as a surfactant protein

3. A method according to any of the preceding claims,
wherein the contact lens is a hydrogel contact lens

4. A method according to any of the preceding claims,
wherein the liquid comprises a polymer formulation.

5. A method according to claim 4, wherein the polymer formulation forms polymer fibre that deposits onto the contact lens to form a thin coating of polymer fibre on at least a proportion of the contact lens, structurally reinforcing the lens.

6. A method according to claim 5, wherein the thin coating is deposited onto the periphery of the contact lens.

7. A method according to claim 4, 5 or 6, wherein the polymer formulation forms polymer fibre of a polymer selected from the group consisting of collagen, polylactide, 2-hydroxyethylmethacrylate, and polyethylene oxide.

8. A method according to any of the preceding claims wherein active ingredient is sprayed onto fibre as it is formed or deposited on the contact lens.

9. A method according to any of the preceding claims wherein the liquid forms fibre and the method further comprises modifying at least one of the fibre and the polymer during flight or after deposition.

10. A method according to claim 9, wherein the polymer is a side-chain modified polymer which is cross-linkable by ultra violet light and the modifying comprises exposing the fibre to ultra violet light during flight and/or after deposition.

11. A method according to claim 9 or 10, wherein part of the deposited fibre is masked so that only an unmasked area is subject to the modification.

12. A method according to any of the preceding claims, further comprising packaging the modified eye covering in a hermetically sealed package.

## Patentansprüche

1. Verfahren zum Modifizieren einer Augenbedeckung in Form einer Kontaktlinse, welches das Positionieren der Augenbedeckung nahe einer elektrohydrodynamischen Zerkleinerungseinrichtung umfasst, die eine Flüssigkeitsversorgung mit einem Auslass und einen elektrischen Feldgenerator umfasst, und welches das Aktivieren der Einrichtung umfasst, so dass Flüssigkeit, die aus dem Auslass austritt, einem elektrischen Feld ausgesetzt wird, das die Flüssigkeit veranlasst, wenigstens einen elektrisch geladenen Strahl zu bilden, der dann wenigstens eines aus einer elektrisch geladenen Faser, Faserfragmenten und Tröpfchen bildet, wobei die Flüssigkeit einen aktiven Inhaltsstoff mitführt.

2. Verfahren gemäß Anspruch 1, wobei der aktive Inhaltsstoff aus der Gruppe bestehend aus einem Antibiotikum, einem schmerzstillenden Mittel, einem entzündungshemmenden Mittel, einem Wachstumsfaktor wie einem Leberzellenwachstumsfaktor, einem epidermalen Wachstumsfaktor zum Fördern des epithelialen Zellwachstums während des Heilungsprozesses und/oder einem phoshpholipiden Netzmittel wie einem Netzmittelprotein ausgewählt wird.

3. Verfahren gemäß irgendeinem der vorhergehenden Ansprüche, wobei die Kontaktlinse eine Hydrogelkontaktlinse ist.

4. Verfahren gemäß irgendeinem der vorhergehenden Ansprüche, wobei die Flüssigkeit eine Polymerformulierung umfasst.

5. Verfahren gemäß Anspruch 4, wobei die Polymerformulierung Polymerfasern bildet, die sich auf der Kontaktlinse ablagert, um eine dünne Beschichtung aus Polymerfasern auf wenigstens einem Anteil der Kontaktlinse zu bilden, wobei sie die Linse strukturell verstärkt.

6. Verfahren gemäß Anspruch 5, wobei die dünne Beschichtung auf der Peripherie der Kontaktlinse abgelagert wird.

7. Verfahren gemäß Anspruch 4, 5 oder 6, wobei die Polymerformulierung Polymerfasern aus einem Polymer bildet, das aus der Gruppe bestehend aus Kollagen, Polylactid, 2-Hydroxyethylmethacrylat und Polyethylenoxid ausgewählt wird.

8. Verfahren gemäß irgendeinem der vorhergehenden Ansprüche, wobei ein aktiver Inhaltsstoff auf Fasern gesprüht wird, während sie auf der Kontaktlinse gebildet oder abgelagert werden.

9. Verfahren gemäß irgendeinem der vorhergehenden Ansprüche, wobei die Flüssigkeit Fasern bildet und das Verfahren ferner das Modifizieren wenigstens eines aus Fasern und Polymer während des Fluges oder nach der Ablagerung umfasst.

10. Verfahren gemäß Anspruch 9, wobei das Polymer ein Seitenkettenmodifikationspolymer ist, das mit ultraviolettem Licht quervernetzbar ist, und wobei das Modifizieren umfasst, die Fasern während des Fluges und/oder nach der Ablagerung ultraviolettem Licht auszusetzen.

11. Verfahren gemäß Anspruch 9 oder 10, wobei ein Teil der abgelagerten Fasern so maskiert wird, dass nur ein unmaskierter Bereich der Modifikation unterworfen wird.

12. Verfahren gemäß irgendeinem der vorhergehenden Ansprüche, das ferner das Verpacken der modifizierten Augenbedeckung in eine hermetisch versiegelte Verpackung umfasst.

## Revendications

1. Procédé de modification d'un revêtement oculaire sous la forme d'une lentille de contact, lequel procédé comprend le positionnement du revêtement oculaire proche d'un dispositif de fragmentation électrohydrodynamique comprenant une alimentation de liquide avec une bouche de sortie et un générateur de champ électrique et l'activation du dispositif de sorte que le liquide provenant de la bouche de sortie soit soumis à un champ électrique poussant le liquide à former au moins un jet électriquement chargé qui forme ensuite une fibre électriquement chargée, des fragments de fibre et/ou des gouttelettes qui se déposent sur le revêtement oculaire, dans lequel le liquide transporte un principe actif.

2. Procédé selon la revendication 1, dans lequel le principe actif est choisi dans le groupe constitué par un antibiotique, un analgésique, un anti-inflammatoire, un facteur de croissance tel que le facteur de croissance des hépatocytes, un facteur de croissance épidermique pour favoriser la croissance des cellules épithéliales pendant le processus de cicatrisation et/ou un tensioactif phospholipidique tel qu'une protéine tensioactive.

3. Procédé selon l'une quelconque des revendications précédentes, dans lequel la lentille de contact est une lentille de contact de type hydrogel.

4. Procédé selon l'une quelconque des revendications précédentes, dans lequel le liquide comprend une formulation de polymère.

5. Procédé selon la revendication 4, dans lequel la formulation de polymère forme une fibre de polymère qui se dépose sur la lentille de contact pour former un revêtement fin de fibre de polymère sur au moins une partie de la lentille de contact, renforçant structurellement la lentille.

6. Procédé selon la revendication 5, dans lequel le revêtement fin est déposé sur la périphérie de la lentille de contact.

7. Procédé selon la revendication 4, 5 ou 6, dans lequel la formulation de polymère former une fibre de polymère à partir d'un polymère choisi dans le groupe constitué par le collagène, un polylactide, le méthacrylate de 2-hydroxyéthyle et un oxyde de polyéthylène.

8. Procédé selon l'une quelconque des revendications précédentes, dans lequel le principe actif est pulvérisé sur la fibre lorsqu'elle est formée ou déposée sur la lentille de contact.

9. Procédé selon l'une quelconque des revendications précédentes, dans lequel le liquide forme une fibre et le procédé comprend en outre la modification de la fibre et/ou du polymère pendant le trajet ou après le dépôt.

10. Procédé selon la revendication 9, dans lequel le polymère est un polymère à chaîne latérale modifiée qui est réticulable par lumière ultraviolette et la modification comprend l'exposition de la fibre à la lumière ultraviolette pendant le trajet et/ou après le dépôt.

11. Procédé selon la revendication 9 ou 10, dans lequel une partie de la fibre déposée est masquée de sorte que seulement une zone non masquée soit soumise à la modification.

12. Procédé selon l'une quelconque des revendications précédentes, comprenant en outre l'emballage du revêtement oculaire modifié dans un emballage hermétiquement scellé.
